Europäisches Patentamt

European Patent Office

Office européen des brevets

(11) Publication number: **0 289 297 B1**

## EUROPEAN PATENT SPECIFICATION

(45) Date of publication of patent specification: **17.03.93**    (51) Int. Cl.5: **C07C 213/08**, C07C 215/76

(21) Application number: **88303825.9**

(22) Date of filing: **28.04.88**

(54) **Improved hydrogenation process for preparing 4-aminophenol.**

(30) Priority: **29.04.87 US 43784**

(43) Date of publication of application:
**02.11.88 Bulletin 88/44**

(45) Publication of the grant of the patent:
**17.03.93 Bulletin 93/11**

(84) Designated Contracting States:
**AT BE CH DE ES FR GB IT LI LU NL SE**

(56) References cited:
**DE-A- 1 643 255        FR-A- 1 559 841
GB-A- 713 622          US-A- 2 780 647
US-A- 2 947 781        US-A- 4 051 187**

(73) Proprietor: **NORAMCO, INC.
1440 Olympic Drive P.O. Box 1652
Athens Georgia 30603(US)**

(72) Inventor: **Kao, James Tzu Fen
7, Candlewood Drive
Princeton Junction, NJ 08550(US)**
Inventor: **Raff II, Dwight Everette
1202 Waterford Road
West Chester, PA 19380(US)**

(74) Representative: **Jones, Alan John et al
CARPMAELS & RANSFORD 43 Bloomsbury
Square
London, WC1A 2RA (GB)**

Rank Xerox (UK) Business Services
(3.10/3.5x/3.0.1)

## Description

This invention relates to a process for preparing 4-aminophenol (p-aminophenol; PAP) from nitrobenzene by catalytic hydrogenation of nitrobenzene to phenylhydroxylamine followed by rearrangement to p-aminophenol without isolation of the intermediate product and is more particularly concerned with improving the efficiency of the reaction by improving the selectivity and the rate of hydrogenation.

### Background of the Invention

The present invention involves an improved, more cost efficient, way of carrying out a known process of preparing 4-aminophenol (p-aminophenol; PAP) from nitrobenzene. PAP is used as an intermediate in the manufacture of acetaminophen (APAP, p-acetaminophenol, N-acetyl p-amino phenol, paracetamol, N-(4-Hydroxyphenyl) acetamide), so any process improvements which lower costs are commercially significant.

In particular, the present invention utilizes the type of process described in Spiegler U.S. Patent No. 2,765,342 and in Benner U.S. Patent No. 3,383,416, (which patents are incorporated herein by reference).

The aforesaid Benner patent first discusses earlier known techniques for the production of para-aminophenol by the catalytic hydrogenation of nitrobenzene as taught in various U.S. patents, e.g., U.S. Patent Nos. 2,198,249 and 2,765,342, and then goes on to disclose a process for the production of para-aminophenol which comprises reducing nitrobenzene with hydrogen in a reaction zone at a temperature of from about 60° to 120°C. in the presence of from about 5 to 20% by weight of aqueous sulfuric acid and a metal-containing catalyst selected from the group consisting of platinum, palladium and mixtures thereof wherein the reduction of the nitrobenzene is interrupted prior to completion to yield a reaction product mixture containing a sufficient amount of unreacted nitrobenzene to form an immiscible layer of nitrobenzene containing the catalyst suspended therein and a separate aqueous layer containing para-aminophenol, and separating the aqueous layer from the nitrobenzene layer containing the catalyst.

The aforesaid Benner patent gives details of operable and preferred reactants, equipment, and reaction conditions e.g. catalyst, reaction temperature, reaction pressure, agitation of reaction mixture requirements, wetting agent, etc. which are necessary so that reasonable yields of p-aminophenol are obtained in a reasonable reduction (hydrogenation) time.

While the specific Benner patent examples are of a batch process, there is discussion of how to operate the process on a continuous basis. The process of the instant invention can be operated on a batch or a continuous basis also.

Sathe U.S. Patent 4,176,138 teaches a method for preparing p-aminophenol, with aniline as a by-product, by the catalytic hydrogenation of nitrobenzene in an acidic reaction medium containing dimethyldodecylamine sulfate.

The p-aminophenol obtained by the Benner patent is a crude p-aminophenol. U.S. Patent Nos. 4,137,562 and 3,694,500 teach processes which are used for purifying the crude p-aminophenol (PAP) which is to be used in making APAP (acetaminophen).

GB. 713,622 teaches a method for preparing p-aminophenol, by the catalytic hydrogenation of nitrobenzene in an aqueous solution containing from 1 to 25% by weight of sulfuric acid at a temperature from 50 to 145°C.

### Summary of the Present Invention

It has now been found that the process of the aforesaid Benner U.S. Patent 3,383,416 for making 4-aminophenol can be carried out at a faster rate of hydrogenation, thereby achieving a great improvement in performance index [expressed as grams 4-aminophenol (g PAP) produced per minute per liter of reaction volume] of 0.2 and higher, as contrasted to the prior art figure of about 0.1 or lower. This higher performance index increases throughput, and thus reduces capital requirement and ultimately production costs. There is no loss of selectivity with this increased rate, and the selectivity actually increases i.e. the proportion of product to by-product improves (contrary to the teachings of Spiegler U.S. 2,765,342). Accordingly, the present invention provides a process for the synthesis of 4-aminophenol by the reduction of nitrobenzene with hydrogen and a hydrogenation catalyst in a sulfuric acid solvent, which comprises pretreating the sulfuric acid with hydrogen peroxide. In the present invention in a preferred embodiment the increased rate of hydrogenation is obtained by pretreating the aqueous sulfuric acid with hydrogen peroxide solution thereby to destroy catalyst poisoning agents, then introducing the hydrogen into the vapor space part of the reactor while stirring the reaction mixture vigorously at a power input of at least $1.97 \text{Js}^{-1}\text{l}^{-1}$ (0.01 horsepower per gallon) using a turbine impeller which is placed at about 40-60% of liquid depth measured from the center of the impeller. The use of hydrogen peroxide pretreated sulfuric acid combined with the agitator design and conditions are believed to be novel for use in this type of process of making PAP, as is the high performance index obtained without altering the optimum product to byproduct ratio.

Detailed Description of the Invention

The Reagents:

In carrying out the process of the present invention to prepare 4-aminophenol via the catalytic hydrogenation of nitrobenzene with an ensuing Bamberger rearrangement of the resulting phenylhydroxyl amine, both the sulfuric acid and nitrobenzene starting materials must be free of catalyst poisons.

Even the use of high quality commercial-grade sulfuric acid may cause problems, e.g., the use of Dupont Electrolyte grade (low iron) 93% sulfuric acid, resulted in complete deactivation of the catalyst and a reaction rate of zero even with extensive nitrogen sparging. This behavior was also observed to varying degrees with different lots of 96%, analyzed reagent grade sulfuric acid from Baker Chemical Company.

It was found that when the sulfuric acid was treated with hydrogen peroxide prior to use, even the above commercial acid (Dupont Electrolyte grade) gives excellent results. This method of removal of catalyst poisons from the sulfuric acid is conducted as follows. A small amount, e.g., about 1.0% by weight, of hydrogen peroxide solution (we prefer a 30% solution, but other strengths can be used) is added to the sulfuric acid, which is then stirred to remove catalyst poisons for a sufficient hold period, i.e., until gas bubbling ceases and the color of the sulfuric acid becomes water clear. The exact amount and strength of hydrogen peroxide used is not critical and can be varied if desired. The exact duration of treatment time required for complete destruction of sulfur dioxide or other potential catalyst poisons will vary depending on the grade of the sulfuric acid used. Oxygen gas is evolved as the hydrogen peroxide is consumed and from 2 to 4 hours are normally required before gas evolution ceases, the yellow color of the acid decreases to a water clear solution, and the sulfuric acid is ready for use in the process of the present invention.

The use of high quality commercial grade nitrobenzene which is free of thiophene and other sulfur-containing compounds gives excellent results in the process of the present invention.

An effective surfactant is also vital to a successful reaction. The Spiegler and the Benner patents each list various wetting agents, such as quaternary ammonium compounds, which can be used. Sathe U.S. 4,176,138 teaches the use of Dodecyldimethyl amine sulfate, which works well here. Lonza Barquat CME-35%, which is N-cetyl-N-ethyl-morpholinium ethosulfate in water, and Witco Emcol CC-55 [polypropoxy quaternary ammonium acetate] have also been tested with good results in the process of the present invention.

The usable catalysts for purposes of the present invention are platinum on carbon catalysts. We prefer to use a 5% platinum on carbon, dry pack catalyst, such as for example the carbon support available from Engelhard Industries as CP-86, which has a surface area of 850 square meters per gram and a mesh size with 90% of the particles smaller than 25 microns, 10% smaller than 5 microns, and a mean size of 10 microns, which works well.

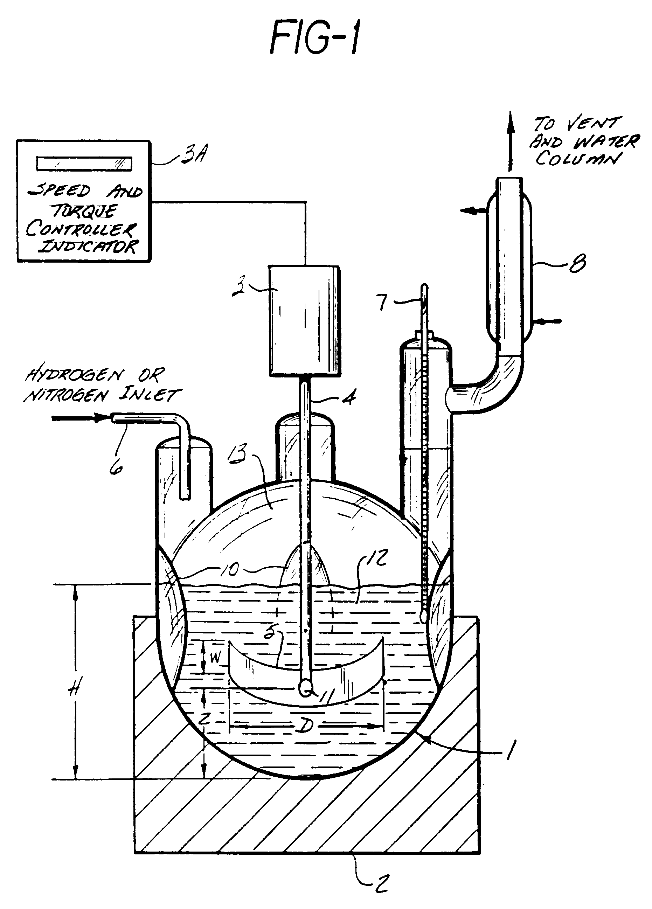

Using the reagents described above, typical experiments were conducted according to the procedure described below in Example 1. The equipment in which the reaction of Example 1 is conducted, is as disclosed in Figure 1.

Figure 1 is a schematic drawing of the reactor 1 and agitator used to carry out the hydrogenation (reduction) process of the present invention. The reactor 1 is a five liter, three neck, four-baffled round bottom flask (i.e. a Morton flask) equipped with a heating mantle 2, overhead mechanical stirring motor 3 which stirring motor is controlled by a controller 3A, which also reads out the speed and torque. The stirring motor 3 drives a stirrer 4, having a curved blade impeller 5, at its lower end. There is a inlet for gas feed 6, i.e. for hydrogen gas, or nitrogen gas leading into one of the necks of the three neck flask.

The other neck of the flask contains a thermometer 7 and a condenser 8 leading to a vent and water column. The four baffles 10 (one is not shown) are indentations equally spaced around the Morton style flask to give greater agitation and promote mixing when the contents are stirred.

The placement of the impeller 5 is measured from its midpoint 11. The flask has a liquid space 12 and a vapor space 13. In Fig 1:H is Liquid Depth, D is Impeller Internal Diameter, W is Impeller Width, Z is Impeller Depth.

Example 1 - General Procedure

A five liter, three neck, four-baffled round bottom flask (hydrogenator) equipped with a heating mantle, overhead mechanical stirring motor [the stirrer motor is controlled by a controller which has both speed and torque readouts and controls] driving a curved blade impeller (Fig. 1), and an inlet for gas feed into the vapor space is charged with 1700 ml of deionized water at an initial temperature of between 20°C and 23°C. Next, 217 g of hydrogen peroxide pretreated 93% sulfuric acid (made as described previously) is added to the system with moderate agitation. The acid addition generates a fairly strong exotherm, raising the temperature of the solution to between 36°C and 38°C. This provides a convenient point to start heating the system to the desired reaction temperature of 94°C. Taking advantage of the acid's heat of solvation to start this temperature increase will lead to savings in time and power consumption. A variac setting of 80 V gives heating at a suitable rate. Next, for the initial run in a series, 271g of nitrobenzene is added. Addition of the nitrobenzene normally decreases the system temperature by about 1°C. Preferably, the reduction of nitrobenzene takes place at an elevated temperature of 95 ± 10°C. For runs in a series using recycled catalyst, 40-50g nitrobenzene would be recycled with the catalyst to aid handling and the charge of fresh nitrobenzene would be reduced accordingly. 2.25ml of dodecyldimethyl amine or other surfactant is added and the system closed. A nitrogen purge with a flow rate of about 100 ml/min is commenced at this point to remove oxygen and any trace volatile catalyst poisons from the system. A constant positive pressure is maintained with a twelve inch water column in the gas exit bubbler. This is equivalent to a moderate, positive pressure of about 26 mmHg above atmosphere.

The agitation rate is increased to 700 rpm or higher and the nitrogen purge continued. Exceedingly vigorous agitation is very important. The power input was calculated from torque and speed readings. Adjustment of speed was made to achieve the desired power input, which is $1.97Js^{-1}l^{-1}$ (0.01 horsepower per gallon) or higher. For a five inch impeller, a speed of 700 rpm gives a power input of $4.34Js^{-1}l^{-1}$ (0.0225) (See Table I). Agitator depth is another critical parameter. For a given power input, maximum gas-liquid interfacial area is generated with the agitator depth equal to 50% of the liquid depth. For practical purposes, the reaction rate is directly proportional to the power input and agitator position. After maintaining the initial nitrogen purge for a sufficient period of time to displace any oxygen in the reactor e.g. ten minutes, 1.40g of dry packed 5% platinum on carbon catalyst is added through an emerging stream of nitrogen, rinsed in with 25ml deionized water, the system resealed, and the 100ml/min nitrogen purge continued for a period to be sure there is no oxygen in the system e.g. for a further 10 minutes. During the period when reagents are being charged to the system, the temperature must be monitored carefully to ensure that it does not exceed 90°C. The heating variac should be adjusted, if necessary, to maintain the temperature below this point.

After completion of the nitrogen purge, addition of hydrogen gas begins. The initial demand can be up to 500ml/min and the gas source must be able to maintain a positive system pressure at all times while meeting this flow rate. A major explosion may result if the system is allowed to generate a partial vacuum and suck air back into the reactor. The hydrogenation is exothermic and a mild exotherm which raises the temperature of the system by about 4°C occurs as the reaction begins. The reaction is allowed to proceed to 75-85% conversion [This is indicated by the hydrogen uptake.] so that the catalyst remains sufficiently wetted by the unreacted nitrobenzene. [In the prior art Benner U.S. Patent 3,383,416 or Sathe U.S. Patent 4,176,138, this step would take 5-7 hours. Here this is achieved in 2-3 hours.]

To terminate the hydrogenation reaction a final nitrogen purge is used. Initially, up to 500ml/min of nitrogen may be required to maintain the system at positive pressure. The flow rate should gradually be reduced to 100ml/min and agitation stopped. Increase the variac setting as needed to maintain a temperature of about 85°C. The aqueous layer [which contains the crude 4-aminophenol (PAP)] is pumped under nitrogen to a 3 liter jacketed flask (decanter) with bottom valve. The reactor jacked is hooked into a circulating bath at 80°C. A nitrogen atmosphere is maintained throughout the work-up procedure because the reaction mixture quickly colors if exposed to air.

[The purification procedure to be described below is the subject matter of pending European patent application EP-A-0 289 298 filed even date herewith and is included here by reference for better understanding of the process of the present invention.]

The pH of the aqueous layer is adjusted to 4.6-4.8 with liquid ammonia. About 80ml (54g) are normally required. It is then extracted 4-7 times with 300 ml(260.1g) of toluene to remove dissolved impurities e.g. aniline, nitrobenzene and oxydianiline (ODA). The pH must be adjusted as the extraction proceeds because it will tend to drop as impurities are extracted into the organic layer. After the extraction cycle is completed 28.6g Anderson AX-21 activated carbon is charged. This material is 65% wet (with water) to aid its handling characteristics and thus a net of 10.0g is used. 5.0g of sodium hydrosulfite are also charged here for

4

decolorization. The activated carbon charcoal is removed via filtration through a standard 12.5cm Buchner filter using a #3 Whatman filter paper. The carbon cake is rinsed twice with 100g aliquots of hot deionized water and discarded. The filtrate and wash are transferred back to the flask. After a nitrogen atmosphere is reestablished over the reaction mixture, the pH is adjusted to 7.2 with liquid ammonia. About 30ml (20.2g) are normally required. The system is slowly cooled to 0°C over a period of 1.5-2 hours and then held for 1 hour with the pH maintained at 7.2.

4-aminophenol is isolated by vacuum filtration, rinsed twice with two 200g aliquots of cold 1% sodium hydrosulfite solution and sucked dry on a frit for several minutes. The material can be acetylated at this point to make APAP, or dried at 50°C in vacuo overnight. The dried 4-aminophenol was white, and weighed 139g. The purity of 4-aminophenol as analyzed by the HPLC method is over 99%. This represents 68% isolated yield based on the nitrobenzene reacted. The unreacted nitrobenzene which contains the catalyst, can be recycled.

Examples 2-4 - Specific Batch-Type Examples

A number of experiments were run using the general procedure described in Example 1 above, but changing one or more of the reaction conditions, as shown in the following Table 1 - Impeller Design and Power Calculation, to obtain the results there shown.

## Table 1 – Impeller Design and Power Calculation

| Reactor | Abb. | Unit | Example 2 | Example 3 | Example 4 |
|---|---|---|---|---|---|
| Impeller Size | | cm (Inch) | 10·2 (4) | 12·7 (5) | 5·1 (2) |
| Reactor Size | | Liter | 5 | 5 | 2 |
| Volume (Liquid) | | Liter | 2.10 | 2.10 | 0.84 |
| Vessel ID | T | cm (In) | 21·2 (8.35) | 21·2 (8.35) | 10·2 (4) |
| Impeller ID | D | cm (In) | 10·2 (4) | 12·3 (4.85) | 5·8 (2.27) |
| Impeller Width | W | cm (In) | 0.8 | 2·5 (1) | 1·2 (0.48) |
| Baffles | B | | Yes | Yes | Yes |
| No. of Baffles | | | 4 | 4 | 4 |
| No. of Impeller Blades | | | 2 | 2 | 2 |
| Liquid Depth | H | cm (In) | 10·6 (4.18) | 10·6 (4.18) | 10·2 (4.00) |
| Impeller Depth | Z | cm (In) | 9·3 (3.68) | 6·0 (2.36) | 5·0 (1.97) |
| Parameter | T/D | | 2.09 | 1.72 | 1.76 |
| Parameter | W/D | | 0.20 | 0.21 | 0.21 |
| Parameter | H/D | | 1.04 | 0.86 | 1.76 |
| Parameter | Z/D | | 0.92 | 0.49 | 0.87 |
| Impeller Placement | Z/H | % | 88% | 57% | 49% |

### Experimental Values

| | Abb. | Unit | Example 2 | Example 3 | Example 4 |
|---|---|---|---|---|---|
| Speed | | RPM | 700 | 700 | 1500 |
| Torque | | $\mu$Nm (Oz-In) | 84,700 (12) | 155,400 (22) | 144,800 (20.5) |
| No Load Torque | | $\mu$Nm (Oz-In) | 28,200 (4) | 28,200 (4) | 28,200 (4) |
| Net Torque | | $\mu$Nm (Oz-In) | 56,500 (8) | 127,100 (18) | 116,500 (16.5) |
| Power Input | | Js$^{-1}$ (HP/Gal) | 1·4 (1.00E-02) | 4·43 (2.25E-02) | 21·9 (1.11E-01) |
| $H_2$ Flow | | CC/Min | 201 | 660 | 380 |
| Productivity Index | | G/L/Min | 0.09 | 0.29 | 0.42 |

In Table I the second column "Abb" gives the abbreviations for the terms in the first column, which terms are shown in the Fig. 1 diagram

In the columns to the right are three Examples 2, 3 and 4, which show the use of different impeller sizes, placements and speeds. The impeller configuration is also indicated by the parameters. In Table I, Example 2 is representative of the prior art conditions as far as the power input and impeller depth are concerned, while Examples 3 and 4 are the results of the present invention.

### Example 5 - Continuous Operation

In a continuous operation, the hydrogenator reactor would be charged as in Example 1 described above, and the reaction started and proceeded with until the desired conversion is achieved. The reaction mixture would be pumped out continuously to the decanter while reactants were pumped in continuously at

EP 0 289 297 B1

the previously mentioned proportions of Example 1. The unreacted nitrobenzene, which contains catalyst, will be separated at the bottom of the decanter and will be pumped back continuously to the hydrogenator. After a desired amount of the aqueous reaction mixture, i.e., a volume of aqueous layer equivalent to a batch run as in Example I, is accumulated in the decanter, the reaction can be interrupted by introducing nitrogen into the hydrogenator and stopping all pumps. The reaction mixture in the decanter can then be worked up as in Example 1.

**Claims**

1. A process for the synthesis of 4-aminophenol by the reduction of nitrobenzene with hydrogen and a hydrogenation catalyst in a sulfuric acid solvent, which comprises pretreating the sulfuric acid with hydrogen peroxide.

2. A process according to claim 1 which additionally comprises stirring the reduction reaction mixture vigorously to attain a performance index, expressed as grams of 4-aminophenol produced per minute per liter of reaction volume, of 0.2 or higher for the reduction reaction.

3. The process of claim 2, wherein the reaction mixture is stirred vigorously at a power input of at least $1.97 Js^{-1}l^{-1}$ (0.01 horsepower per gallon).

4. The process of claim 2 or claim 3, wherein the reaction is conducted in a reactor fitted with a turbine impeller and said impeller is placed at about 40-60% of liquid depth in the reaction zone.

5. The process according to any one of the preceding claims, wherein the hydrogen peroxide is mixed with the sulfuric acid until bubbling ceases.

6. The process according to any one of the preceding claims, wherein the hydrogen peroxide is added to the sulfuric acid in an amount of about 1% by weight of the sulfuric acid.

7. The process according to any one of the preceding claims, wherein the hydrogen peroxide is mixed with the sulfuric acid until the sulfuric acid is water clear.

8. The process of any one of the preceding claims, wherein the reaction is conducted in a reaction zone which is not completely filled so that there is both a vapor space and a lower liquid space in the reaction zone.

9. The process of any one of the preceding claims, wherein the reduction of nitrobenzene takes place at an elevated temperature of 95 ± 10°C.

**Patentansprüche**

1. Verfahren zur Synthese von 4-Aminophenol durch die Reduktion von Nitrobenzol mit Wasserstoff und einem Hydrierungskatalysator in Schwefelsäure als Lösungsmittel, das die Vorbehandlung der Schwefelsäure mit Wasserstoffperoxid umfasst.

2. Verfahren nach Anspruch 1, das zusätzlich das starke Rühren des Reduktions-Reaktionsgemisches umfasst, um einen Wirkungsindex für die Reduktionsreaktion von 0,2 oder darüber zu erreichen, angegeben in Gramm 4-Aminophenol, die in einer Minute je Liter Reaktionsvolumen gebildet werden.

3. Verfahren nach Anspruch 2, bei dem das Reaktionsgemisch stark mit einem Leistungsbedarf von mindestens 1,97 $Js^{-1}l^{-1}$ (0,01 PS je Gallone) gerührt wird.

4. Verfahren nach Anspruch 2 oder 3, bei dem die Reaktion in einem Reaktor durchgeführt wird, der mit einem Turbinenrührer ausgerüstet ist und bei dem der genannte Turbinenrührer in etwa 40 bis 60 % der Flüssigkeitstiefe in der Reaktionszone angebracht ist.

5. Verfahren nach einem der vorstehenden Ansprüche, bei dem das Wasserstoffperoxid mit der Schwefelsäure vermischt wird, bis die Blasenbildung aufhört.

7

6. Verfahren nach einem der vorstehenden Ansprüche, bei dem das Wasserstoffperoxid zu der Schwefelsäure in einer Menge von etwa 1 Gew.-% der Schwefelsäure gegeben wird.

7. Verfahren nach einem der vorstehenden Ansprüche, bei dem das Wasserstoffperoxid mit der Schwefelsäure vermischt wird, bis die Schwefelsäure wasserklar ist.

8. Verfahren nach einem der vorstehenden Ansprüche, bei dem die Reaktion in einer Reaktionszone durchgeführt wird, die nicht vollständig gefüllt ist, so daß sowohl ein Dampfraum wie auch ein niedigerer Flüssigkeitraum in der Reaktionszone vorhanden ist.

9. Verfahren nach einem der vorstehenden Ansprüche, bei dem die Reduktion des Nitrobenzols bei einer höheren Temperatur von 95 +/-10°C erfolgt.

## Revendications

1. Procédé pour la synthèse d'amino-4-phénol par la réduction du nitrobenzène avec de l'azote et un catalyseur d'hydrogénation dans un acide sulfurique solvant qui comprend le pré-traitement de l'acide sulfurique avec du peroxyde d'hydrogène.

2. Procédé selon la revendication 1 qui comprend additionnellement l'agitation vigoureuse du mélange réactionnel de réduction pour atteindre un indice de performance, exprimé en grammes d'amino-4-phénol produit par minute par litre de volume de la réaction, de 0,2 ou plus pour la réaction de réduction.

3. Procédé de la revendication 2, où le mélange réactionnel est soumis à agitation vigoureuse à une énergie d'entrée d'au moins 1,97 $Js^{-1}l^{-1}$ (0,01 cheval par gallon).

4. Procédé de la revendication 2 ou la revendication 3, où la réaction est entreprise dans un réacteur pourvu d'une roue de turbine et ladite roue est placée à peu près à 40-60% de profondeur du liquide dans la zone de réaction.

5. Procédé selon l'une quelconque des revendications précédentes, où le peroxyde d'hydrogène est mélangé à l'acide sulfurique jusqu'à ce qu'il n'y ait plus de bouillonnements.

6. Procédé selon l'une quelconque des revendications précédentes, où le peroxyde d'hydrogène est ajouté à l'acide sulfurique en une quantité d'environ 1% en poids de l'acide sulfurique.

7. Procédé selon l'une quelconque des revendications précédentes, où le peroxyde d'hydrogène est mélangé à l'acide sulfurique jusqu'à ce que l'acide sulfurique soit limpide comme l'eau.

8. Procédé selon l'une quelconque des revendications précédentes, où la réaction est entreprise dans une zone réactionnelle qui n'est pas complètement remplie de manière qu'il y ait à la fois un espace de vapeur et un espace inférieur de liquide dans la zone réactionnelle.

9. Procédé selon l'une quelconque des revendications précédentes, où la réduction du nitrobenzène a lieu à une température élevée de 95±10°C.

# FIG-1